Europäisches Patentamt

(19) European Patent Office

Office européen des brevets

(11) Publication number: 0 043 963
A1

(12) EUROPEAN PATENT APPLICATION

(21) Application number: 81104988.1

(22) Date of filing: 26.06.81

(51) Int. Cl.³: C 07 C 41/03
C 07 C 43/11, C 08 G 65/28
B 01 F 17/42, C 11 D 1/66

(30) Priority: 30.06.80 US 164008

(43) Date of publication of application:
20.01.82 Bulletin 82/3

(84) Designated Contracting States:
AT BE DE FR GB IT LU NL SE

(71) Applicant: UNION CARBIDE CORPORATION
270, Park Avenue
New York, N.Y. 10017(US)

(72) Inventor: Decker, Quintin William
1006 Sand Hill Drive
Saint Albans 25177 West Virginia(US)

(72) Inventor: Fields, Robert Riley
609 Dupont Avenue
Nitro 25143 West Virginia(US)

(72) Inventor: Marcus, Erich
1005 Knob Rd.
Charleston 25314 West Virginia(US)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) Improved process for ethoxylation of broad-range primary alcohols.

(57) A process is provided for the preparation of stable, nonionic surface active agents which comprises in a first stage, reacting ethylene oxide with a primary monohydric alcohol having a range of chain lengths containing 10 to 20 carbon atoms in the presence of an acid catalyst for the time necessary to obtain an adduct containing 1 to 6 moles of ethylene oxide and then in a second stage, reacting ethylene oxide with the neutralized and washed reaction product from the first stage in the presence of an alkaline catalyst for the time necessary to prepare a stable, liquid nonionic surface active agent.

## BACKGROUND OF THE INVENTION

This invention relates to the preparation of nonionic surface active agents and, more particularly, to a process for the preparation of improved nonionic surface active agents from mixtures of monohydric alcohols containing from 10 to 20 carbon atoms with up to about 30 percent by weight thereof being alcohols containing from 16 to 20 carbon atoms.

It has long been the practice to prepare surface active agents by the addition of ethylene oxide to long-chain monohydric alcohols. However, adducts prepared by the addition of ethylene oxide to primary alcohols with substantial proportions thereof containing more than about 16 carbon atoms have been found to have higher pour points than is generally satisfactory, the products prepared therefrom containing solids or forming a precipitate during storage, when formulated with other ingredients, or when diluted with water. It is therefore normal practice to use monohydric alcohols feed stocks with different chain length ranges which are chosen with reference to their ultimate use. Primary aliphatic monohydric alcohols derived from vegetable oils and fats will contain a range of chain lengths which can range from $C_{10}$ to $C_{20}$ and beyond. Separation of these products into different chain length ranges can be costly as well as being wasteful of materials that may not have wide usefulness.

Such ethylene oxide adducts are conventionally prepared by reacting a higher straight-chain or

-2-

branched-chain primary monohydric alcohol of a selected chain length range with ethylene oxide at elevated temperatures generally in the presence of an alkaline catalyst or, for certain applications, in the presence of an acidic catalyst. While the compounds thus made are effective surface active agents, it would be desirable from an economic standpoint to be able to produce compounds from mixtures of monohydric alcohols containing a wider range of chain lengths, particularly alcohols containing 16 and more carbon atoms, which exhibited lower pour points and were free of solids or the formation of a precipitate.

It has also been suggested in the past as, for example, disclosed in U.S. Patent 2,870,220 to Carter, that a two-stage method for the ethoxylation of alcohols can be used to convert secondary and primary alkanols having 10 to 17 carbon atoms into highly efficient surface active agents. In this process, the reaction of ethylene oxide with secondary and primary alkanols is carried out during the first stage in the presence of an acidic catalyst such as boron trifluoride and then, after removal of the acid catalyst and any unreacted alkanol, reacts the adduct so produced with ethylene oxide during a second stage in the presence of an alkaline catalyst. The patent, however, is primarily directed to the ethoxylation of secondary alkanols and while it involves alkanols having a relatively wide range of chain lengths, there is no disclosure of its suitability for use with primary monohydric alcohols having chain lengths containing more than 17 carbon atoms or with substantial amounts of long-chain monohydric alcohols. Moreover, a separation procedure for unreacted alkanol is required in addition to the two ethoxylation stages.

## SUMMARY OF THE INVENTION

In accordance with the present invention there is provided a process for the preparation of stable (does not form a precipitate or become cloudy on storage) nonionic surface active agents by converting primary monohydric alcohols which contain a range of chain lengths having 10 to 20 or more carbon atoms which comprises reacting ethylene oxide with primary monohydric alcohols up to about 30 percent by weight of which contain from about 16 to 20 carbon atoms, in the presence of an acid catalyst for the time necessary to obtain an adduct containing 1 to 6 moles of ethylene oxide, neutralizing and washing said reaction mixture and then reacting the same with ethylene oxide in the presence of an alkaline catalyst. The nonionic surface active agents prepared thereby are stable, clear liquids which do not form a precipitate after extended periods of storage or when mixed with other conventional formulating ingredients.

## DESCRIPTION OF THE INVENTION

In accordance with the present invention, primary monohydric alcohols having 10 to 20 carbon atoms can be readily converted with good yields into products which are highly efficient surface active agents that do not form a precipitate during storage or when formulated by a novel two-stage process. In the first stage thereof, the monohydric alcohol and ethylene oxide are reacted in the presence of an acidic catalyst under conditions favoring the initial reaction of ethylene oxide with the monohydric alcohol. This reaction mixture, after removal of the acidic catalyst and byproducts, is reacted with ethylene oxide in the presence of an alkaline catalyst which can be an alkali metal alcoholate of the initial alcohol or the corresponding alcoholate of the adduct made during the first stage of the process. These catalysts can be made in situ by reacting the neutralized product of the first reaction stage with an alkali metal, alkali metal oxide or hydroxide.

In accordance with a preferred embodiment of this invention, ethylene oxide is reacted in a first reaction stage with a straight-chain or branched-chain higher primary monohydric alcohol or mixtures thereof having from 10 to about 20 carbon atoms with up to about 30 percent by weight thereof containing 16 or more carbon atoms in the presence of an acidic catalyst. The acidic catalyst used in the first stage of the process conveniently can be one of the well known class of the Friedel-Crafts type reaction catalysts, such as the fluorides and chlorides of boron, aluminum, iron, tin and titanium, and complexes of such halides with ethyl ether. Sulfuric acid and phosphoric acid also are effective.

Preferably the ethylene oxide is slowly added during several hours to a monohydric alcohol having from 0.02% to 0.2% or more of its weight of the acidic catalyst present therein while maintaining the reaction mixture at a temperature within the range from about 20°C to about 80°C, and preferably, at about 50°C, and at pressures from around atmospheric to 100 psi gauge. A stream of the ethylene oxide is passed into the alcohol and reacted therewith in a molar ratio within the range from 1:1 to 6:1, and preferably, from 2:1 to 4:1. This reaction is continued until all of the ethylene oxide added has reacted with the alcohol.

The reaction mixture is then neutralized, washed, and preferably dried to remove the acid catalyst and by-products. Commonly, a 10% caustic solution can be used to neutralize and wash the reaction product.

The residue is monoalkyl ethers of ethylene glycol and lower polyethylene glycols, generally having average molecular weights in the range from 300 to 500.

The residue of monoalkyl ethers of ethylene glycol and polyethylene glycols has then mixed therein between about 0.05 and about 0.5 weight percent thereof, and preferably about 0.1 and 0.2 weight percent based on final ethoxylate, of an alkaline catalyst, preferably an alkali metal alcoholate of the adduct present in said residue from the first-stage reaction or of the initial monohydric alcohol.

The alcoholate can be made in situ by reacting the residue from the first-stage reaction with a powdered caustic alkali, or with an alkali metal or an alkali metal alcoholate of a low molecular weight alkanol such as methanol while heating the mixture to elevated temperatures in the range from about 90°C to about 180°C. The reaction desirably is conducted in an atmosphere of nitrogen until all of the caustic alkali, alkali metal or the equivalent has reacted with the reaction mixture.

Then ethylene oxide is slowly added to the reaction mixture while maintaining the temperature within the range from about 90°C to about 180°C and preferably from about 100°C to about 150°C until a 1.0 weight percent aqueous solution of the resultant product has a cloud point within the range from 10°C to 100°C. As more ethylene oxide reacts the product becomes more soluble in water, and the cloud point rises concurrently. The cloud point is that temperature at which a 1.0% aqueous solution of the product becomes cloudy.

Thus, stable, highly efficient nonionic surface active agents from mixtures of monohydric alcohols having from 10 to about 20 atoms are readily prepared. The surface active agents are stable liquids which do not form a precipitate or become cloudy after extended periods of storage or when mixed with conventional formulating agents. Formulations prepared therewith also are clear when mixed with water. Moreover, the novel two-stage process of the invention can be carried out without the need to remove unreacted alcohol from the reaction mixture before progressing to the second stage of the process, which is clearly an operating advantage over two-stage ethoxylation processes that were heretofore suggested.

Among the monohydric alcohols that have been found suitable in accordance with the practice of the invention are straight- or branched-chain primary aliphatic alcohols having from 10 to about 20 carbon atoms with up to about 30 per cent by weight thereof containing from about 16 to 20 carbon atoms. Suitable monohydric alcohols are the primary alcohols obtained from the hydrogenation of vegetable or animal fatty acids such as coconut, palm kernel, and tallow fatty acids, the commercially available alcohols prepared thereby comprising alcohol mixtures. Also suitable are linear and branched primary alcohols and alcohol mixtures such as are produced by the "oxo" reaction of linear olefins having from 10 to 20 carbon atoms. Exemplary suitable primary aliphatic alcohols are n-decanol, n-undecanol, n-dodecanol, n-tridecanol, n-tetradecanol, n-pentadecanol, n-hexadecanol, n-heptadecanol, n-octadecanol, n-nonadecanol, 2-methyl-1-nonanol, 2-methyl-1-undecanol, 2-methyl-1-dodecanol, 2-methyl-1-tetradecanol and the like, and mixtures thereof.

The invention will become more clear when considered together with the following examples which are set forth as being merely illustrative of the invention and which are not intended in any manner, to be limitative thereof. Unless otherwise indicated, all parts and percentages are by weight.

## Example 1

### Part A

A mixture of 2490 grams (11.74 moles) of a broad-range commercially available primary alcohol mixture (molecular weight 212) containing between 10 and 20 carbon atoms with 20 percent thereof being $C_{16}$-$C_{20}$ alcohols and 2.4 milliliters of boron trifluoride monoetherate were charged in a nitrogen atmosphere to a two-gallon stirred reactor having a circulating pump. The reactor was pressurized with nitrogen to a pressure of 10 psig, the circulating pump was turned on, and the reaction mixture was heated to 60°C. The nitrogen pressure was then adjusted to 20 psig and 1557 grams (35.24 moles) of ethylene oxide were slowly added to the reactor at 60 psig over a twenty-minute period. After cooking out the reaction for 1-1/4 hours, the reactor was cooled to ambient temperature and its contents (3996 grams) were discharged under a nitrogen atmosphere.

A portion of the reaction product (1100 grams) was placed in a 5-liter steam-jacketed flask and washed with 200 ml of a one percent aqueous solution of sodium hydroxide while vigorously stirring the contents at 95°C for 15 minutes. The contents were allowed to settle and the water layer was withdrawn. The organic layer was washed twice with water at 95°C, and after separation of the layers, the organic layer (1181 grams) was determined to have a pH of 6.8.

The organic layer was then charged to a 3-liter flask and stripped at a temperature of 100°C and 5 mm Hg pressure to remove the water therefrom. Overhead material and cold traps held 107 grams and 1064 grams of reaction mixture residue remained. The residue was found to contain 4.8 percent of unreacted alcohol.

Two milliliters of a 50 percent $NaBH_4$ solution and 4 grams of 90 percent potassium hydroxide were added to the reaction mixture residue which was heated to 100°C while reducing the pressure to 5 mm Hg to remove water. After heating for one hour, analysis showed the reaction mixture to contain 0.03 percent water.

The dried reaction mixture (1067 grams) was charged to the two-gallon reactor with a nitrogen purge and pressurized to 10 psig with nitrogen. The circulation pump was turned on and the reaction mixture was heated to 146°C at which time the pressure was adjusted to 20 psig with nitrogen. 529 grams of ethylene oxide (12.1 moles) were added to the reactor at 60 psig over a fifteen-minute period and the reaction mixture was cooked out for 15 minutes. The reactor was then cooled to ambient temperature and its contents were discharged in a nitrogen-purged flask. The reaction product was then neutralized to a pH of 6.8 with acetic acid and stripped at 80°C and a pressure of 5 mm Hg to remove unreacted ethylene oxide. The product was a clear liquid which was determined to have a cloud point (1% solution) of 53°C, a hydroxyl number of 106 and a molecular weight of 530.

The reaction product was then used to prepare three formulations having the following proportion of ingredients

| Formulation | A | B | C |
| --- | --- | --- | --- |
| Nonionic Surfactant | 64 | 70.1 | 70 |
| Triethanolamine | 21 | 11.7 | 11 |
| Ethanol | 6 | 10.6 | 13 |
| Water | 9 | 7.6 | 6 |

After storage of each of these compositions for 30 days at 25°C no precipitate was found to have formed.

Part B

Using the alcohol mixture of Part A, 1700 grams (8.02 moles) thereof were charged to a 3-liter, 3-necked, round-bottom flask equipped with a stirrer, thermowell, nitrogen purge, and heating mantle. The alcohol was heated to 50°C with stirring and purged with nitrogen for 15 minutes. Flake 90 percent potassium hydroxide (12.4 grams, 0.25 percent) was then added and the mixture was heated at 95°C at 5 mm Hg for 1 hour to remove water. The dried alcohol (1699 grams) was transferred in a nitrogen atmosphere to a two-gallon reactor equipped with a stirrer and a

11.

12633

0043963

circulation pump. The reactor was pressurized to 10 psig using nitrogen, the circulation pump was turned on, and the reaction mixture was heated to 144°C. After adjusting the pressure to 20 psig with nitrogen, 2526 grams (57.34 moles) of ethylene oxide were metered into the reactor at 60 psig over a period of thirty-four minutes. The reaction mixture was cooked out for 10 minutes and then cooled to ambient temperature. After discharging the reaction mixture into a nitrogen-purged flask, the product was neutralized with acetic acid to a pH of 6.8 and stripped at 90°C and a pressure of 5 mm Hg to remove unreacted ethylene oxide.

The reaction product was a liquid having a cloud point (1% solution) of 53°C, a hydroxyl number of 107, and a molecular weight of 522.

The nonionic surface active agent was used in three formulations having the same proportion of ingredients as described in Part A, above. After storage for 30 days at 25°C, a precipitate was found to have formed in each of the three formulations.

Example II

Using the apparatus and procedure of Example I, Part A, a nonionic surface active agent was prepared from an initial charge of 1007 grams of a primary aliphatic monohydric alcohol (4.71 moles) and 1 ml. of boron trifluoride monoetherate. During the first stage of the procedure, 623 grams (14.13 moles) of ethylene oxide were added to the

12.

reaction mixture and, during the second stage of the procedure, an additional 761 grams (17.3 moles) of ethylene oxide were added to the reaction mixture residue of 1420 grams.

The primary monohydric alcohol used in this example was prepared from a commercial primary linear alcohol containing from 12 to 14 carbon atoms which is available under the trademark designation of ALFOL 1214 from Continental Oil Company to which was added 20 percent by weight of an alcohol cut having from 16 to 20 carbon atoms. The alcohol mixture had a molecular weight of 214.

The nonionic surface active spent product that was prepared was determined to have a cloud point (1% solution) of 51°C, a hydroxyl number of 104, and a molecular weight of 538.

The reaction product was used to prepare three formulations using the same proportion of ingredients described in Example 1. Each of these formulations exhibited no precipitate formation after storage for 30 days at 25°C.

Example 3

A mixture of 485 grams (2.31 moles) of a broad-range primary alcohol and 0.7 ml. of boron trifluoride monoetherate were charged to a 1.5- gallon stirred reactor under a nitrogen atmosphere. Using the procedure of Example 1, Part A, a two-stage reaction process was carried out with 302 grams (6.86 moles) of ethylene oxide being added to the reaction mixture during the first stage and 383 grams (8.70 moles) of ethylene oxide being added to 653 grams of reaction mixture residue during the second stage.

The broad range primary alcohol used as the starting material in this example was prepared from a mixture of $C_{12}$ to $C_{15}$ primary alcohols (60% branched, 40% normal isomers) available commercially under the tradename LIAL-125 from Liquichemica Italia to which was added 20 percent of an alcohol cut containing 16 to 20 carbon atoms. The alcohol starter had a molecular weight of 210.

The reaction product of the two-stage process of this example was a clear liquid which was determined to have a cloud point (1% solution) of 47°C, a hydroxyl number of 97, and a molecular weight of 579.

Three formulations were prepared with the reaction product of this example having the proportion of ingredients described in Example 1. After storage for 30 days at 25°C no precipitate was found to have formed in any of these formulations.

PATENTANWÄLTE

WUESTHOFF - v. PECHMANN - BEHRENS - GOETZ

EUROPEAN PATENT ATTORNEYS

EP-54 922          - 15 -

**0043963**

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING.; DIPL.-WIRTSCH.-ING. RUPERT GOETZ

D-8000 MÜNCHEN 90
SCHWEIGERSTRASSE 2

TELEFON: (089) 66 20 51
TELEGRAMM: PROTECTPATENT
TELEX: 5 24 070

## PATENT  CLAIMS

1. A process for preparing stable nonionic surface active agents which comprises in a first stage reacting ethylene oxide with a mixture of primary monohydric alcohols in the presence of an acid catalyst consisting of Friedel-Crafts type catalysts, sulfuric acid, and phosphoric acid for the time necessary to prepare an adduct of said alcohol containing 1 to 6 moles of ethylene oxide and in a second stage reacting ethylene oxide with the neutralized and washed reaction product from the first stage in the presence of an alkaline catalyst for the time necessary to prepare a stable, liquid nonionic surface active agent.

2. The process of claim 1 in which said mixture of primary alcohols have between 10 and about 20 carbon atoms.

3. The process of claim 1 or 2 in which up to about 30 percent by weight of said mixture of primary alcohols contain 16 to about 20 carbon atoms.

4. The process of claim 1 to 3 in which said primary alcohols are reacted with between about 2 and 4 moles of ethylene oxide per mole of alcohol.

5. The process of claim 1 to 4 in which the adduct reacted with ethylene oxide in said second stage contains unreacted alcohol.

6. A stable, nonionic surface active agent which is prepared using the process of claim 1 to 5.

0043963

European Patent
Office

EUROPEAN SEARCH REPORT

Application number

EP 81 10 4988

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | DE - A - 2 030 131 (CONTINENTAL OIL CO.)<br>* Pages 1-15 *<br>& GB - A - 1 280 563<br><br>-- | 1-6 | C 07 C 41/03<br> 43/11<br>C 08 G 65/28<br>B 01 F 17/42<br>C 11 D 1/66 |
| | DE - A - 1 518 958 (GENERAL ANILINE & FILM CO.)<br>* Pages 1-6 *<br>& GB - A - 1 125 935<br><br>-- | 1 | |
| D | US - A - 2 870 220 (C.A. CARTER)<br>* Claims 1-4 *<br><br>---- | 1 | TECHNICAL FIELDS SEARCHED (Int. Cl.³)<br><br>C 07 C 41/03<br>C 08 G 65/28 |

CATEGORY OF
CITED DOCUMENTS

X: particularly relevant
A: technological background
O: non-written disclosure
P: intermediate document
T: theory or principle underlying
   the invention
E: conflicting application
D: document cited in the
   application
L: citation for other reasons

&: member of the same patent
   family,
   corresponding document

The present search report has been drawn up for all claims

| Place of search | Date of completion of the search | Examiner |
|---|---|---|
| The Hague | 14-10-1981 | VERHULST |

EPO Form 1503.1  06.78